# EUROPEAN PATENT SPECIFICATION

(11) **EP 1 874 363 B1**
(45) Date of publication and mention of the grant of the patent: **06.05.2009**
(21) Application number: 06710097.4
(22) Date of filing: 16.03.2006
(51) Int. Cl.: A61L 15/28, A61L 15/46

(54) **PHOTOSTABLE WOUND DRESSING MATERIALS AND METHODS OF PRODUCTION THEREOF**
PHOTOSTABILE WUNDVERBANDSMATERIALIEN UND HERSTELLUNGSVERFAHREN DAFÜR
MATERIAUX PHOTOSTABLES POUR PANSEMENTS ET LEURS PROCEDES DE FABRICATION

(30) Priority: 26.04.2005 GB 0508431
(43) Date of publication of application: 09.01.2008
(73) Proprietor: ETHICON INC., Somerville, NJ 08876 (US)
(72) Inventor: CULLEN, Breda, Mary, Skipton BD23 1HR (GB); SILCOCK, Derek, Walter, Skipton BD23 1QP (GB); BOYLE, James, Glasgow G44 3PD (GB)
(74) Representative: James, Anthony Christopher W.P.
(86) International application number: PCT/GB2006/000935
(87) International publication number: WO 2006/114565

(56) References cited:
- EP-A- 0 901 795
- WO-A-02/43743
- WO-A-20/04024197
- WO-A-20/04112850
- GB-A- 2 402 880

## Description

The present invention relates to wound dressing materials comprising complexes formed between anionic polysaccharides, such as oxidized regenerated cellulose (ORC), and silver, and to the methods of production and uses thereof for the treatment of wounds.

Anionic polysaccharides such as alginates, hyaluronic acid and its salts, and oxidized celluloses such as oxidized regenerated cellulose (ORC) are known for use in wound dressing materials. Alginates and ORC are hemostatic when applied to a wound, and ORC has been shown to promote the healing of chronic wounds such as dermal ulcers.

It is known to incorporate antimicrobial materials into wound dressings. Silver is used as an antimicrobial in various forms, including colloidal metal particles, silver salts and other silver compounds. The silver can be coated on, or incorporated into the fibers, polymers, textiles and adhesive components used in the fabrication of wound dressings.

WO2004/024197 describes wound dressing materials comprising complexes (salts) of anionic polysaccharides with ionic silver. The materials preferably comprise about 0.1 to 2wt.% of silver. This silver concentration range was found to combine antimicrobial action with low cytotoxicity and enhanced wound healing.

WO03/028762 and WO2004/112805 describe antimicrobial compositions comprising an antimicrobial substance such as silver, and at least one compound which inhibits microbial resistance to the antimicrobial. The resistance inhibitor may for example comprise an antioxidant, or a compound that interacts with the microbial cell wall to allow silver into the cell and/or to disrupt the ion pump mechanisms that remove silver from microbial cells. WO04/112805 suggests the possibility of using a number of compounds, including ascorbate for this purpose.

However, the comparative data in WO03/028762 show that ascorbate is not effective for reducing microbial resistance to triclosan.

A problem with materials containing silver salts or compounds is that they are often sensitive to light, which can result in undesirable discoloration of the silver-containing material and other drawbacks. Efforts have been made to overcome this technical problem.

For example, WO02/43743 describes wound dressings comprising a silver salt of an anionic polysaccharide, and further comprising a substance to improve the photostability of the silver salt. The stabilising substance may include ammonia, ammonium salts, thiosulphates, chlorides and/or peroxides. In one preferred embodiment, the stabilising agent is aqueous ammonium chloride.

The wound dressing materials of WO2004/024197 are made by a process that comprises forming an aqueous dispersion comprising ORC fibers which have been partially ion-exchanged with silver. The dispersion preferably further comprises silver-free ORC fibers and collagen fibers. The dispersion is acidified with acetic acid, and is dried by freeze-drying or solvent drying to form a sponge material suitable for application to a wound. The products develop an attractive pink colour on exposure to light due to the photochemical reduction of silver in the material. Nevertheless, it would be desirable to provide wound dressing materials that do not discolor, or that discolor only to a minimal extent, on exposure to light.

The present inventors have found that by acidifying the aqueous dispersions used in the methods of WO2004/024197 with ascorbic acid (or certain other medically acceptable acids) instead of acetic acid, it is possible to obtain a freeze-dried silver/ ORC/collagen sponge having a stable white or near-white colour. It is not necessary to add supplemental stabilising agents to prevent photochemical discoloration of these materials.

Ascorbic acid has previously been suggested for use in swelling homogenised collagen to a premix or gel suitable for freeze-drying to form a collagen sponge (US-A-5565210). It has also been proposed to add ascorbic acid and its salts to wound dressing materials to act as an acid buffer system for maintaining stable wound pH (EP-A-0901795). However, the present invention is based on the surprising finding that ascorbic acid can apparently stabilise ionic silver against photochemical degradation in the wound dressing materials of WO2004/024197, without loss of other desirable properties.

In a first aspect, the present invention relates to a method of preparing an antimicrobial sponge material for medicinal use, comprising the steps of
a) treating an anionic polysaccharide with a solution of a silver salt to produce a complex of the anionic polysaccharide with silver,
b) dispersing said complex in aqueous ascorbic acid to form an acidified dispersion, followed by
c) freeze-drying or solvent-drying the dispersion to form the sponge material.

As discussed above, silver containing materials are typically sensitive to light and discolor upon exposure to light. The materials made in accordance with the present invention have greater stability to light than the materials made in accordance with WO2004/024197. Preferably, the materials obtained or obtainable by the method of the present invention do not undergo a substantial colour change upon exposure to light, i.e. they are substantially photostable. More preferably, the materials obtained or obtainable by the method of the present invention do not undergo any colour change upon exposure to light. It is surprising that ascorbic acid, which functions as an antioxidant in nature, should apparently also be effective to reduce photochemical reduction of silver in the wound dressing materials.

Preferably, the anionic polysaccharide is a polycarboxylate. Suitable anionic polysaccharides include alginates, hyaluronates, pectins, carrageenans, xanthan gums, sulfated dextrans, cellulose derivatives such as carboxymethyl celluloses, and oxidized celluloses. Preferably, the anionic polysaccharide comprises an oxidized cellulose, and it may consist essentially of the oxidized cellulose.

The term "oxidized cellulose" refers to any material produced by the oxidation of cellulose, for example with dinitrogen tetroxide. Such oxidation converts primary alcohol groups on the saccharide residues to carboxylic acid groups, forming uronic acid residues within the cellulose chain. The oxidation generally does not proceed with complete selectivity, and as a result hydroxyl groups on carbons 2 and 3 are occasionally converted to the keto form. These keto units introduce an alkali-labile link, which at pH 7 or higher initiates the decomposition of the polymer via formation of a lactone and sugar ring cleavage. As a result, oxidized cellulose is biodegradable and bioabsorbable under physiological conditions.

The preferred oxidized cellulose for use in the present invention is oxidized regenerated cellulose (ORC) prepared by oxidation of a regenerated cellulose, such as rayon. It has been known for some time that ORC has haemostatic properties. ORC has been available as a haemostatic fabric called SURGICEL (Registered Trade Mark of Johnson & Johnson Medical, Inc.) since 1950. This product is produced by the oxidation of a knitted rayon material.

Preferably, the anionic polysaccharide is insoluble in water at pH≤7. Preferably, the anionic polysaccharide has a molecular weight greater than about 20,000, more preferably greater than about 50,000. Preferably, the anionic polysaccharide is in the form of a film, or fibers having length greater than 1 mm.

In the inventive method, the anionic polysaccharide is treated with a solution of a silver salt to produce a complex of the anionic polysaccharide with silver. The term "complex" refers to an intimate mixture at the molecular scale, preferably with ionic or covalent bonding between the silver and the polysaccharide. The complex preferably comprises a salt formed between the anionic polysaccharide and Ag⁺.

Preferably, the silver salt solution is an aqueous solution. The solution should be prepared in a quantity sufficient to provide the desired silver concentration in the resulting complex.

Anionic polysaccharides behave as an ion exchanger and will pull out of solution the silver cation of any silver salt that is passed over them. The by-product of this exchange is an acid from the salt and by using a salt of a weak organic acid, a weak acid is produced which does no damage to the polysaccharide. Using salts of strong acids such as sodium chloride or sodium sulfate produces hydrochloric acid or sulfuric acid by-products respectively, and these strong acids can cause damage such as depolymerization of the polysaccharide.

When using silver salts of weak acids, the silver ion is exchanged for a proton on the polysaccharide and part of the salt is converted to weak acid. The mixture of acid and salt in the solution results in a buffered solution which maintains a fairly constant pH and controls the degree of neutralisation. An equilibrium reaction is established whereby the silver ions are bound to the acid portion of the polysaccharide and also to the salt molecules. This partitioning of the silver ions prevents the neutralisation of the polysaccharide from going to completion.

Using a stoichiometric amount of, for example, silver acetate brings about a 65-75% degree of neutralisation of the carboxylic acid groups on an oxidized cellulose polymer. This control of pH by creating a self generating buffered solution and the use of methanol to control the swelling of the material, leads to a partially neutralised material in which the physical properties, e.g. tensile strength and shape of the polysaccharide, are preserved.

The amount of silver salt used is generally about equal to or up to twice the stoichiometric amount of carboxylic acid content of the polysaccharide. Alternatively, a second charge of a stoichiometric amount of silver salt can be used if the reaction is recharged with fresh solvent and salt after the first charge reaches a constant pH. The material with elevated pH is then washed to remove the excess silver salt and ions therefrom.

The length of time that the anionic polysaccharide is subjected to the solution is a period sufficient to incorporate the desired concentration of silver into the complex. Preferably, the anionic polysaccharide is treated with the solution for between 1 and 120 minutes. In some embodiments, the treatment time is 10, 20, 30, 40, 50, 60 or more minutes. Generally, the length of time necessary will depend on the anionic polysaccharide used and can be easily determined by the skilled person.

Preferably, the solution and the anionic polysaccharide are protected form light throughout the treatment.

Preferably the amount of silver in the complex is from 0.1% to 50% by weight based on the weight of the anionic polysaccharide, more preferably from 1 % to 40%, still more preferably from 2% to 30% by weight, and most preferably from 5% to 25%.

The second step of the method of the invention requires that the silver-containing complex is dispersed in an aqueous solution of ascorbic acid. Typically, the ascorbic acid is buffered to pH from 2 to 5, preferably pH 3 to 4, and the total ascorbate concentration is from 0.5%w/v to 5%w/v, preferably 1 %w/v to 2%w/v. The acid is suitable to provide the desired texture in the final, dried sponge product. Preferably, the dispersion further contains other polymeric components. For example, the dispersion may comprise one or more further anionic polysaccharides, e.g. anionic polysaccharides that have not been complexed to silver. These polysaccharides may include any one or more of the anionic polysaccharides listed in above as being suitable for complexation with silver.

Preferably, the further anionic polysaccharide is a polycarboxylate, more preferably oxidised cellulose. Most preferably, the further anionic polysaccharide is oxidized regenerated cellulose (ORC).

The additional polymeric component may additionally or alternatively comprise any other medically acceptable polymers, such as for example collagens, celluloses, regenerated celluloses such as rayon, non-anionic cellulose derivatives such as hydroxyethyl cellulose, and starch derivatives.

The collagen may be selected from native collagens such as Types I, II or III native collagens, natural fibrous collagen, atelocollagen, partially hydrolysed collagens such as gelatin, regenerated collagen and combinations thereof. Preferably, the collagen is not freeze dryed before mixing with the silver-anionic polysaccharide complex and the unmodified anionic polysaccharide.

Preferably, all polymeric components of the dispersion are fully bioabsorbable, whereby the product sponge can be made fully bioabsorbable.

Step (c) of the method according to this aspect of the invention comprises freeze-drying or solvent-drying the dispersion. Freeze-drying comprises the steps of freezing the dispersion, followed by evaporating the solvent from the frozen dispersion under reduced pressure. Suitably, the method of freeze-drying is similar to that described for a collagen-based sponge in US-A-2157224. Solvent drying comprises freezing the dispersion, followed by immersing the frozen dispersion in a series of baths of a hygroscopic organic solvent such as anhydrous isopropanol to extract the water from the frozen dispersion, followed by removing the organic solvent by evaporation. Methods of solvent drying are described, for example, in US-A-3157524.

In certain embodiments the process may further comprise treating the dispersion, or the dried material, with a cross-linking agent such as epichlorhydrin, carbodiimide, hexamethylene diisocyanate (HMDI) orglutaraldehyde. Alternatively, cross-linking may be carried out dehydrothermally. The method of cross-linking can markedly affect the final product. For example,HMDI cross-links the primary amino groups on collagen, whereas carbodiimide cross-links carbohydrate on the ORC to primary amino groups on the collagen.

The methods of the present invention are preferably used to produce photostable versions of the bioabsorbable sponges according to WO2004/024197. As already noted, WO2004/024197 discloses the production of wound dressing materials comprising a complex of an anionic polysaccharide with silver (a silver-ORC complex). These dressings are produced by freeze-drying or solvent-drying an aqueous dispersion of a silver/ORC complex, unmodified milled ORC and collagen.

The aqueous dispersion is made with a sufficient amount of water acidified with ascorbic acid to obtain a total solids content of preferably 0.5% to 4%, preferably 1% to 2%.

Preferably, the dispersion contains from 0.1wt% to 100wt% of the silver-anionic polysaccharide complex, more preferably from 0.1wt% to 5wt.%, for example from 0.2wt.% to 2wt.%, based on the dry weight of the polymeric components in the dispersion. The amount of silver in the final product after drying is preferably from 0.1wt% to 3wt.%, more preferably from more than 0.1wt% to 1wt.%, and for example from 0.2wt.% to 0.6wt.%, typically 0.3wt.%. Lesser amounts of silver could give insufficient antimicrobial effect. Greater amounts of silver could give rise to antiproliferative effects on wound healing cells.

Preferably the amount of collagen in the dispersion is from 10% to 90% by weight based on the dry weight of the wound dressing materials, more preferably from 25% to 75%. Most preferably, the amount of collagen in the mixture is 55%.

The mixture may further comprise anionic polysaccharides that have been complexed to therapeutically effective metal ions other than silver, for example bismuth, copper, nickel, zinc, manganese, magnesium, gold, or mixtures thereof. Preferably, the amounts of such polysaccharides complexed to other metals is from 0.001 to 10wt.% of the dressing, more preferably from 0.01 to 1wt% of the dressing. Preferably, the amounts of said other metals is from 10 to 10000ppm, more preferably from 50 to 1000ppm in the dressing.

In particular embodiments, the polymeric components of the mixture to be freeze dried comprise (and may consist essentially of) a mixture of (a) silver-anionic polysaccharide complex, (b) unmodified anionic polysaccharide and (c) collagen for example in a dry weight ratio of about 1%/44%/55%.

In certain embodiments of the first aspect of the invention, at least a portion of the collagen in the mixture is also complexed with silver. This can be achieved by treating the collagen with a solution of a silver salt. The silver salt may for example be silver acetate or silver nitrate at a concentration of 0.01 molar to 1 molar. The treatment is preferably carried out at a pH of from 5 to 9. It is thought that the silver complexes primarily to the nitrogen-containing side chains of the collagen amino acids, in particular to lysine, hydroxylysine, asparagine, glutamine and arginine. The silver could also bind to the sulfhydryl groups of methionine and cysteine residues, where present, and to carboxyl groups of aspartate and glutamate.

Preferably the amount of silver in the collagen complex is from 0.01 to 30% by weight based on the weight of the collagen, more preferably from 0.1 % to 20%, more preferably from 2% to 10% by weight. Preferably, the amount of silver-collagen complex in the freeze dried bioabsorbable sponge is from 0.1 to 10 wt.%, more preferably from 0.1 to 2wt.%.

In a second aspect, the present invention provides a photostabilized medical sponge material comprising silver (I) ions, wherein the material is obtained or obtainable by the method of the first aspect of the invention. The material suitably further comprises ascorbate ions, typically in an amount of at least 1% based on the dry weight of the material, more suitably at least 10%.

In a third aspect, the present invention provides a photostabilized medical sponge material comprising an anionic polysaccharide complexed with silver (I) ions, wherein the sponge further comprises ascorbate, and the sponge has a substantially white colour that is stable against discoloration on exposure to light. The ascorbate is typically present in an amount of at least 1% based on the dry weight of the material, more suitably at least 10%, for example up to 50%.

The term "stable against discoloration on exposure to light" signifies that there is substantially no color change detectable to the eye (as determined by color card comparisons) upon exposure of the material to natural daylight (not direct sunlight) at ambient temperature and in air for at least 24 hours, preferably at least 10 days, and more preferably at least one month.

It has been found that the photostable freeze dried sponges of the invention do not discolor on exposure to light, unlike freeze dried sponges wherein the sponge structure comprises acetic acid as described in WO2004/024197. It has been found by the inventors that colour of the final product after exposure to light is dependant on the acid used to acidify the aqueous dispersion. Acetic acid (as used in the methods of WO2004/024197) produces a product that goes pink on exposure to light. However, ascorbic acid produces a white product that does not undergo a colour change over time.

In addition to its antimicrobial properties, it was noted in WO2004/024197 that silver ions at low concentrations of 0.1wt.% to 3wt.% in the freeze dried sponge exhibits an enhanced proliferative effect on wound healing cells and therefore is expected to promote wound healing. The present inventors have found that this effect is enhanced in the sponges containing ascorbic acid when compared to that achieved by the freeze dried sponges described in WO2004/024197.

Preferably, the average pore size of the sponges of the invention is in the region of 10-500µm, more preferably 100-300µm. In certain embodiments, the a freeze-dried bioabsorbable sponge according to the present invention is in a form suitable for application to a wound. The wound maybe an acute wound, a chronic wound or an infected wound.

Especially preferred are freeze-dried sponges comprising from 35wt% to 60wt% of ORC, from 60wt.% to 35wt.% of a collagen, and from 0.5% to 5wt.% of an ORC/silver complex, as defined above. In preferred embodiments, the materials comprise from 0.5wt.% to 2wt. % of the ORC/silver complex. In preferred embodiments, the polymeric components of the materials consist essentially of the collagen, ORC, and ORC/silver complex. In preferred embodiments, the the materials consist essentially of the ascorbate, collagen, ORC, and ORC/silver complex.

Preferably, the photostabilized medical sponges of the invention are suitable for use in the preparation of antimicrobial wound dressing materials. The silver confers antimicrobial properties on the wound dressing. As such, in a further aspect, the invention also provides a wound dressing consisting of or comprising the medical sponge according to the second or third aspect of the invention.

In a further aspect, the present invention provides the use of medical sponge according to the second or third aspects of the invention for the preparation of a wound dressing material for the treatment of wounds, especially chronic wounds such as venous ulcers, decubitis ulcers or diabetic ulcers. Preferably, the treatment comprises applying the wound dressing material directly to the surface of the wound.

The wound dressing may be bioabsorbable or non bioabsorbable. The term 'bioabsorbable' as used herein refers to a wound dressing that is fully degraded and absorbed in vivo in the mammalian body.

Preferably, the sponge material is in the form of a sheet, for example a sheet of substantially uniform thickness. The area of the sheet is typically from 1 cm² to 400cm², and the thickness typically from 1 mm to 10mm. Preferably, the material comprises less than 15% by weight of water, more preferably less than 10% by weight of water.

Preferably, the sponge material comprises from 0.1 wt% to 100wt% of the silver-containing complex, more preferably from 0.1wt% to 5wt.%, for example from 0.2wt.% to 2wt.%. The amount of silver in the wound dressing material is from 0.1wt% to 3wt.%, preferably from more than 0.1wt% to 1wt.%, and for example from 0.2wt.% to 0.6wt.%, typically about 0.3wt.%. Lesser amounts of silver could give insufficient antimicrobial effect. Greater amounts of silver could give rise to antiproliferative effects on wound healing cells.

The wound dressing is preferably in sheet form and comprises an active layer of the freeze dried bioabsorbable sponge according to the invention. The active layer would normally be the wound contacting layer in use, but in some embodiments it could be separated from the wound by a liquid-permeable top sheet. Preferably, the area of the active layer is from 1cm² to 400 cm², more preferably from 4cm² to 100cm².

Preferably, the wound dressing further comprises a backing sheet extending over the active layer opposite to the wound facing side of the active layer. Preferably, the backing sheet is larger than the active layer such that a marginal region of width 1 mm to 50 mm, preferably 5mm to 20mm extends around the active layer to form a so-called island dressing. In such cases, the backing sheet is preferably coated with a pressure sensitive medical grade adhesive in at least its marginal region.

Preferably, the backing sheet is substantially liquid-impermeable. The backing sheet is preferably semipermeable. That is to say, the backing sheet is preferably permeable to water vapour, but not permeable to liquid water or wound exudate. Preferably, the backing sheet is also microorganism-impermeable. Suitable continuous conformable backing sheets will preferably have a moisture vapour transmission rate (MVTR) of the backing sheet alone of 300 to 5000 g/m²/24hrs, preferably 500 to 2000 g/m²/24hrs at 37.5 °C at 100% to 10% relative humidity difference. The backing sheet thickness is preferably in the range of 10 to 1000 micrometers, more preferably 100 to 500 micrometers.

The MVTR of the dressing according to the present invention as a whole is lower than that of the backing sheet alone, because the apertured sheet partially obstructs moisture transfer through the dressing. Preferably, the MVTR of the dressing (measured across the island portion of the dressing) is from 20% to 80% of the MVTR of the backing sheet alone, more preferably from 20% to 60% thereof, and most preferably about 40% thereof. It has been found that such moisture vapour transmission rates allow the wound under the dressing to heal under moist conditions without causing the skin surrounding the wound to macerate.

Suitable polymers for forming the backing sheet include polyurethanes and poly alkoxyalkyl acrylates and methacrylates such as those disclosed in GB-A-1280631. Preferably, the backing sheet comprises a continuous layer of a high density blocked polyurethane foam that is predominantly closed-cell. A suitable backing sheet material is the polyurethane film available under the Registered Trade Mark ESTANE 5714F.

The adhesive (where present) layer should be moisture vapour transmitting and/or patterned to allow passage of water vapour therethrough. The adhesive layer is preferably a continuous moisture vapour transmitting, pressure-sensitive adhesive layer of the type conventionally used for island-type wound dressings, for example, a pressure sensitive adhesive based on acrylate ester copolymers, polyvinyl ethyl ether and polyurethane as described for example in GB-A-1280631. The basis weight of the adhesive layer is preferably 20 to 250 g/m², and more preferably 50 to 150 g/m². Polyurethane-based pressure sensitive adhesives are preferred.

Further layers of a multilayer absorbent article may be built up between the active layer of sponge material according to the present invention and the protective sheet. For example, these layers may comprise an apertured plastic film to provide support for the active layer in use.

The dressing may further comprise an absorbent layer between the active layer of sponge material according to the present invention and the protective sheet, especially if the dressing is for use on exuding wounds. The optional absorbent layer may be any of the layers conventionally used for absorbing wound fluids, serum or blood in the wound healing art, including gauzes, nonwoven fabrics, superabsorbents, hydrogels and mixtures thereof. Preferably, the absorbent layer comprises a layer of absorbent foam, such as an open celled hydrophilic polyurethane foam prepared in accordance with EP-A-0541391, In other embodiments, the absorbent layer may be a nonwoven fibrous web, for example a carded web of viscose staple fibers. The basis weight of the absorbent layer may be in the range of 50-500g/m², such as 100-400g/m². The uncompressed thickness of the absorbent layer may be in the range of from 0.5mm to 10mm, such as 1 mm to 4mm. The free (uncompressed) liquid absorbency measured for physiological saline may be in the range of 5 to 30 g/g at 25°. Preferably, the absorbent layer or layers are substantially coextensive with the silver-polysaccharide layer.

The wound facing surface of the dressing is preferably protected by a removable cover sheet. The cover sheet is normally formed from flexible thermoplastic material. Suitable materials include polyesters and polyolefins. Preferably, the adhesive- facing surface of the cover sheet is a release surface. That is to say, a surface that is only weakly adherent to the wound facing surface of the dressing and the adhesive on the backing sheet to assist peeling of the hydrogel layer from the cover sheet. For example, the cover sheet may be formed from a non-adherent plastic such as a fluoropolymer, or it may be provided with a release coating such as a silicone or fluoropolymer release coating.

Preferably, the wound dressings and materials according to the present invention are sterilized. Preferably, they are packaged in a microorganism-impermeable container. Preferably, the sterility assurance level is better than 10⁻⁶. Preferably, the product has been sterilised by gamma-irradiation.

The present invention may be used in a method of treatment of a wound, comprising applying to the wound a wound dressing comprising an effective amount of a medical sponge material according to the invention, whereby said dressing is antimicrobially effective without exhibiting substantial antiproliferative activity against wound healing cells. Preferably, the wound dressing material is also effective to reduce inflammation. The method is especially suitable for treatment of chronic wounds such as venous ulcers, decubitis ulcers or diabetic ulcers.

In a further aspect, the present invention provides the use of ascorbic acid in an antimicrobial material comprising a silver (I) salt of a medically acceptable polyanionic acid to stabilize the material against discoloration on exposure to light.

It will be appreciated that the medical sponges obtainable by the methods of the first aspect of the invention may be used in the products and methods according to any aspect of the invention.

The term "ascorbic acid" encompasses salts, derivatives and analogs of ascorbic acid having similar ability to reduce discoloration of silver (I) containing materials.

The term "comprising" as used herein encompasses "including" as well as "consisting" e.g. a composition "comprising" X may consist exclusively of X or may include something additional e.g. X + Y.

Embodiments of the process and product according to the present invention will now be described further, together with reference examples, as follows.

### Procedure 1

A complex of ORC with silver was prepared as follows.

A SURGICEL cloth (Johnson & Johnson Medical, Arlington) was milled using a rotary knife cutter through a screen-plate, maintaining the temperature below 60°C, to provide a fibrous ORC powder

Silver acetate powder (4.08g) was dissolved in 800mls of de-ionised water. After all the powder had dissolved to form a colourless, clear solution, the ORC milled fibers (5g) were added to this solution. The ORC was then allowed to react for sixty minutes, or less if desired. The solution was then filtered and the fibers were collected and washed with either de-ionised water or alcohol. The silver complex of ORC was then allowed to dry at 37°C overnight or until the fibers were dry. To reduce the darkening of the final product the solution and reacted fibers were protected from the light throughout the reaction.

### Example 1

Freeze-dried coliagen/ORC sponges suitable for use as wound dressing materials according to the invention were prepared as follows.

First, the collagen component was prepared from bovine corium as follows. Bovine corium was split from cow hide, scraped and soaked in sodium hypochlorite solution (0.03% w/v) to inhibit microbial activity pending further processing.

The corium was then washed with water and treated with a solution containing sodium hydroxide (0.2% w/v) and hydrogen peroxide (0.02% w/v) to swell and sterilise the corium at ambient temperature.

The corium splits then underwent an alkali treatment step in a solution containing sodium hydroxide, calcium hydroxide and sodium bicarbonate (0.4% w/v, 0.6% w/v and 0.05% w.v, respectively) at pH greater than 12.2, ambient temperature, and for a time of 10-14 days, with tumbling, until an amide nitrogen level less than 0.24mmol/g was reached.

The corium splits then underwent an acid treatment step with 1% hydrochloric acid at ambient temperature and pH 0.8-1.2. The treatment was continued with tumbling until the corium splits had absorbed sufficient acid to reach a pH less than 2.5. The splits were then washed with water until the pH value of corium splits reached 3.0-3.4.

The corium splits were then comminuted with ice in a bowl chopper first with a coarse comminution and then with a fine comminution setting. The resulting paste, which was made up in a ratio of 650g of the corium splits to 100g of water, as ice, was frozen and stored before use in the next stage of the process. However, the collagen was not freeze-dried before admixture with the ORC in the next stage.

The silver-ORC complex, unmodified milled ORC powder, and the required weight (according to solids content) of frozen collagen paste were then added to a sufficient amount of water acidified with ascorbic acid to obtain a pH value of 3.0 and a total solids content of 1.0%-2.0%, to give a proportion by weight of 1% Silver-ORC + 44% ORC / 55% Collagen

The mixture was homogenized through a Fryma MZ130D homogenizer, progressively diminishing the settings to form a homogeneous slurry. The pH of the slurry was maintained at 2.9-3.1. The slurry temperature was maintained below 20°C, and the solids content was maintained at 1% ± 0.07.

The resulting slurry was pumped to a degassing vessel. Vacuum was initiated for a minimum of 30 minutes, with intermittent stirring, to degas the slurry. The slurry was then pumped into freeze-drier trays to a depth of 25mm. The trays were placed onto freezer shelves where the temperature has been pre-set to -40°C. The freeze-drier programme was then initiated to dry and dehydrothermally crosslink the collagen and ORC to form thick sponge pads.

On completion of the cycle, the vacuum was released, the freeze-dried blocks were removed, and were then split to remove the top and bottom surface layers, and to divide the remainder of the blocks into 3mm-thick pads. The step of splitting the freeze-dried blocks into pads was carried out with a Fecken Kirfel K1 slitter.

Finally, the pads were die-cut to the desired size and shape on a die-cutter, packaged, and sterilised with 18-29 KGy of cobalt 60 gamma-irradiation. Surprisingly, this irradiation does not cause significant denaturation of the collagen, which appears to be stabilised by the presence of ORC. The resulting freeze-dried collagen ORC pads had a uniform, white, velvety appearance. The white color of the pads was stable on exposure to daylight for extended periods. The texture of the pads was soft and flexible.

### Reference Example 2

The method of Example 1 was repeated, but using acetic acid to acidify the slurry, as described in WO2004/024197. The resulting sponge materials were soft and flexible, but discolored to a pink color on exposure to daylight.

### Reference Example 3

The method of Example 1 was repeated, but using citric acid to acidify the slurry. The resulting sponge materials were relatively hard and friable, and discolored to a red/brown color on exposure to natural daylight.

### Reference Example 4

The method of Example 1 was repeated, but using lactic acid to acidify the slurry. The resulting sponge materials were relatively dense and friable. The materials exhibited an off-white (cream) color that was stable on exposure to natural daylight.

### Procedure 2

The antiproliferative effects of the dressings from the above Examples were assessed by the method described in WO2004/024197, as follows.

Prototype extracts were prepared as follows - 1 mg of each wound dressing material to be tested was placed in 1ml of serum free medium and incubated for 24 hours at 37°C under sterile conditions.

Adult human dermal fibroblasts were grown and maintained in DMEM 10% FBS (standard culture medium; Dulbecco's miminal essential medium; foetal bovine serum). These cells were routinely subcultured and used for experimental testing when 95% confluent. Adult human dermal fibroblasts were harvested at 95% confluency and re-seeded in a 96-well microtitre plate (100µl/well) in DMEM + 10% FBS at a cell density of 2.5 x 10⁴ cells/ml. Cells were allowed to adhere to the plate surface for 24 hours in a humidified incubator at 37°C, 5% CO₂. The medium was then removed by aspiration and the cell monolayer washed with serum-free DMEM. Test samples (extracts of each prototype) were then added to the cell monolayer (100µl/well), and 6 replicates of each concentration tested. Serum-containing growth medium (10% FBS in DMEM) was used as a positive control, and serum-free medium was used as a negative control. All samples were incubated with the cells for 48 hours at 37°C, 5% CO₂. After this incubation period the conditioned medium was removed by aspiration and replaced with a labelling solution from a commercial cell proliferation kit (XTT, Cell Proliferation kit II, Cat. No. 1 465 015, obtained from Boehringer Mannheim). Once this solution was added an initial absorbance reading was obtained at 450nm, after which the microtitre plate was incubated at 37°C, 5% CO₂ and the absorbance monitored over 4 hours. The proliferative effect of each prototype was evaluated by comparing the absorbance readings measured against the positive and negative controls.

It was found that the samples made with acetic acid, ascorbic acid and lactic acid strongly promoted fibroblast proliferation. The effect was greatest for the samples made with ascorbic acid and lactic acid. The sample made with citric acid was found to kill the fibroblast cells.

## Claims

1. A method of preparing an antimicrobial sponge material for medicinal use, comprising the steps of:
treating an anionic polysaccharide with a solution of a silver salt to produce a complex of the anionic polysaccharide with silver;
dispersing said complex in aqueous ascorbic acid to form an acidified dispersion, followed by
freeze-drying or solvent-drying the dispersion to form the sponge material.

2. The method of claim 1 wherein the ionic polysaccharide consists essentially of oxidised regenerated cellulose (ORC).

3. The method of any preceding claim, wherein the said dispersion further comprises one or more additional medically acceptable polymeric materials dispersed therein.

4. The method of claim 3, wherein the dispersion further comprises a silver-free ORC and a collagen, and the silver concentration in the dispersion is from 0.1 % to 2% by weight based on total polymeric solids.

5. The method of any preceding claim wherein the dispersion has a pH of from 3 to 4, and a solids concentration of from 0.5% to 2%.

6. A photostabilized antimicrobial sponge material obtained or obtainable by a method according to any of claims 1 to 5.

7. A photostabilized antimicrobial sponge material comprising an anionic polysaccharide complexed with silver (I) ions, wherein the sponge further comprises ascorbate, and the sponge has a substantially white colour that is stable against visible discoloration on exposure to natural daylight for 24 hours at 25°C and in air.

8. A wound dressing comprising an antimicrobial sponge material according to claim 6 or 7.

9. A wound dressing according to claim 8, wherein the wound dressing is sterile and packaged in a microorganism-impermeable container.

10. Use of ascorbic acid in an antimicrobial material comprising a silver (I) salt of a medically acceptable polyanionic acid to stabilize said material against discoloration on exposure to light.

## Patentansprüche

1. Verfahren zur Herstellung eines antimikrobiellen Schwammmaterials für medizinische Verwendung, das die Schritte umfasst:
Behandeln eines anionischen Polysaccharids mit einer Lösung eines Silbersalzes, um einen Komplex des anionischen Polysaccharids mit Silber herzustellen;
Dispergieren des Komplexes in wässriger Ascorbinsäure, um eine angesäuerte Dispersion zu bilden, gefolgt von
Gefriertrocknen oder Lösemitteltrocknen der Dispersion, um das Schwammmaterial zu bilden.

2. Verfahren nach Anspruch 1, wobei das ionische Polysaccharid im wesentlichen aus oxidierter regenerierter Cellulose (ORC) besteht.

3. Verfahren nach einem vorangehenden Anspruch, wobei die Dispersion weiter ein oder mehr zusätzliche medizinisch verträgliche polymere Materialien umfasst, die darin dispergiert sind.

4. Verfahren nach Anspruch 3, wobei die Dispersion weiter eine silberfreie ORC und ein Collagen umfasst und die Silberkonzentration in der Dispersion von 0,1 bis 2 Gew.-% beträgt, bezogen auf die polymeren Feststoffe insgesamt.

5. Verfahren nach einem vorangehenden Anspruch, wobei die Dispersion einen pH von 3 bis 4 und eine Feststoffkonzentration von 0,5% bis 2% besitzt.

6. Photostabilisiertes antimikrobielles Schwammaterial, erhalten oder erhältlich mit einem Verfahren nach einem der Ansprüche 1 bis 5.

7. Photostabilisiertes antimikrobielles Schwammaterial, das ein anionisches Polysaccharid, komplexiert mit Silber(I)-Ionen umfasst, wobei der Schwamm weiter Ascorbat umfasst und der Schwamm eine im wesentlichen weiße Farbe besitzt, die gegen sichtbare Verfärbung bei Einwirkung von natürlichem Tageslicht für 24 Stunden bei 25°C und an Luft stabil ist.

8. Wundverband, der ein antimikrobielles Schwammaterial nach Anspruch 6 oder 7 umfasst.

9. Wundverband nach Anspruch 8, wobei der Wundverband steril und in einem für Mikroorganismen undurchlässigen Behälter verpackt ist.

10. Verwendung von Ascorbinsäure in einem antimikrobiellen Material, das ein Silber(I)-Salz einer medizinisch verträglichen polyanionischen Säure umfasst, um das Material gegen Verfärbung bei Einwirkung von Licht zu stabilisieren.

## Revendications

1. Procédé de préparation d'une mousse antimicrobienne à des fins thérapeutiques, comprenant les étapes consistant à:
➢ traiter un polysaccharide anionique avec une solution d' un sel d'argent pour produire un complexe du polysaccharide anionique avec l'argent ;
➢disperser ledit complexe dans de l'acide ascorbique aqueux pour former une dispersion acidifiée,
puis
➢ lyophiliser ou sécher par solvant la dispersion pour former la mousse.

2. Procédé selon la revendication 1, dans lequel le polysaccharide ionique consiste essentiellement en de la cellulose régénérée oxydée (ORC).

3. Procédé selon l'une quelconque des revendications précédentes, dans lequel ladite dispersion comprend en outre un ou plusieurs matériaux polymères médicalement acceptables supplémentaires dispersés dans celle-ci.

4. Procédé selon la revendication 3, dans lequel la dispersion comprend en outre une ORC sans argent et un collagène, et la concentration en argent dans la dispersion va de 0,1 % à 2 % en poids sur la base des solides polymères totaux.

5. Procédé selon l'une quelconque des revendications précédentes, dans lequel la dispersion présente un pH allant de 3 à 4, et une concentration en solides allant de 0,5 % à 2 %.

6. Mousse antimicrobienne photostabilisée obtenue ou pouvant être obtenue par un procédé selon l'une quelconque des revendications 1 à 5.

7. Mousse antimicrobienne photostabilisée comprenant un polysaccharide anionique complexé avec des ions argent (I), dans laquelle la mousse comprend en outre de l'ascorbate, et la mousse présente une couleur sensiblement blanche qui est Stable vis-à-vis d'une décoloration visible lors d'une exposition à la lumiére du jour pendant 24 heures à 25°C et dans l'air.

8. Pansement comprenant une mousse anti-microbienne selon la revendication 6 ou 7.

9. Pansement selon la revendication 8, dans lequel le pansement est stérile et conditionné dans un contenant imperméable aux microorganismes.

10. Utilisation d'acide ascorbique dans un matériau antimicrobien comprenant un sel d'argent (I) d'un acide poly(anionique) médicalement acceptable pour stabiliser ledit matériau vis-à-vis d'une décoloration lors d'une exposition à la lumière.
